# FASCICULE DE BREVET EUROPEEN

(11) **EP 1 550 431 B1**
(45) Date de publication et mention de la délivrance du brevet: **27.07.2011**
(21) Numéro de dépôt: 04300951.3
(22) Date de dépôt: 23.12.2004
(51) Int. Cl.: A61K 8/37, A61K 8/81, A61Q 1/06

(54) **Composition cosmétique associant un ester éthylénique en configuration trans et une cire hydrocarbonée**
Kosmetische Zusammensetzung mit einem trans-konfigurierten ethylenischen Ester und einem Kohlenwasserstoffwachs
Cosmetic composition with an ethylenic ester in trans-conformation together with a hydrocarbon wax

(30) Priorité: 05.01.2004 FR 0450003
(43) Date de publication de la demande: 06.07.2005
(73) Titulaire: L'Oréal, 75008 Paris (FR)
(72) Inventeur: Lebre, Caroline, 94320, THIAIS (FR); Filippi, Vanina, 75015, PARIS (FR)
(74) Mandataire: Le Coupanec, Pascale A.M.P.

(56) Documents cités:
- EP-A- 1 410 784
- US-A- 4 360 387
- US-A- 4 393 043
- US-A- 5 968 530
- M. Schlossman,ed.: "The chemistry and manufacture of cosmetics. Volume II-Formulating. Ed.3", 2000, Allured Pub. pages 629-657,
- M. Schlossman ed.: "The chemistry and manufacture of cosmetics, Volume III, book 2, edition 3.", 2002, Allured Pub. pages 1104-1107,

## Description

La présente invention vise à proposer des compositions cosmétiques solides dotées de propriétés améliorées en terme d'application, de confort, de résistance mécanique et de stabilité.

Plus particulièrement le domaine de l'invention est celui du maquillage et/ou du soin de la peau et des phanères. D'une manière générale, ces compositions se doivent de donner satisfaction en termes de confort. Plus particulièrement elles doivent être dotées d'une onctuosité suffisante pour procurer à l'application un effet fondant, doux et glissant, tout en étant suffisamment structurées pour être prises au doigt ou pour être conditionnées, par exemple en pot, en stylo ou en stick.

Les compositions de l'art antérieur qui sont faciles à appliquer et crémeuses à l'application manquent souvent de stabilité dans le temps; soit que les huiles qu'elles contiennent migrent à la surface de la composition, qui devient brillante (phénomène d'exsudation ou de déphasage), soit que les cires qu'elles contiennent subissent un phénomène de recristallisation, si bien que la composition devient mate.

En outre, dans le cas des compositions solides, lorsqu'on cherche à augmenter la quantité du dépôt de composition sur les matières kératiniques en un seul passage, ou lorsqu'on souhaite rendre le dépôt plus souple à l'application, la résistance mécanique de la composition devient généralement insuffisante, si bien que la composition casse ou s'écrase au moment où l'utilisateur l'applique et que la composition ne peut plus être utilisée.

Ainsi, il est très difficile d'obtenir une composition cosmétique présentant à la fois de bonnes propriétés d'application (douceur, onctuosité, crémeux), et une structure acceptable, c'est-à-dire résistant notamment à des températures de l'ordre de 38 °C, et conservant un aspect de surface lisse et sans imperfection au cours du temps (sans exsudation, ni recristallisation).

Les inventeurs ont trouvé qu'il est possible d'obtenir une composition dotée à la fois d'une résistance mécanique suffisante et d'une qualité d'application améliorée. Cette composition contient au moins une cire hydrocarbonée ayant un point de fusion relativement élevé et au moins un ester insaturé ayant un point de fusion relativement bas.

Le document US 4 360 387 décrit des compositions cosmétiques contenant un mélange d'huile de jojoba hydrogénée et du beurre de jojoba. Ces compositions présentent cependant le désavantage d'exsuder au cours du temps ou de ne pas être suffisamment crémeuses à l'application.

Les inventeurs ont en effet trouvé qu'en associant à un ester insaturé tel que le beurre de jojoba, une cire hydrocarbonée de point de fusion suffisamment élevé il est possible d'obtenir une composition cosmétique remédiant aux inconvénients cités précédemment.

Plus précisément, la présente invention concerne une composition cosmétique, solide, notamment pour le soin et/ou le maquillage de la peau, des lèvres et/ou des matières kératiniques caractérisée en ce qu'elle associe à au moins un ester d'un acide éthylénique en C16 à C24 et d'un alcool éthylénique en C16 à C24 avec ledit acide et/ou ledit alcool étant en configuration trans, au moins une cire hydrocarbonée de polyéthylène de point de fusion supérieur à 70 °C.

De façon surprenante, l'association d'une cire hydrocarbonée de polyéthylène de haut point de fusion à ce type d'ester permet de conférer à ces compositions cosmétiques, les propriétés mécaniques attendues sans porter préjudice à leurs propriétés en terme de confort. Ainsi, les compositions cosmétiques conformes à l'invention s'avèrent posséder de bonnes propriétés d'application se traduisant notamment par une sensation de fondant, de glissant, d'onctuosité et de douceur.

Les compositions de l'invention présentent également une stabilité mécanique et thermique dans le temps satisfaisante, ce qui est d'autant plus surprenant que les compositions de l'art antérieur, glissantes à l'application, présentent généralement une stabilité insuffisante.

Enfin, les compositions conformes à l'invention ne s'avèrent pas sujettes à des phénomènes de recristallisation au cours du temps, phénomènes qui sont bien entendu préjudiciables en termes d'esthétique, puisqu'ils matifient la surface de la composition. Les compositions présentent également l'avantage de ne pas exsuder au cours du temps.

La présente invention a en outre pour objet l'utilisation de l'association d'au moins un ester d'un acide éthylénique en C16 à C24 et d'un alcool éthylénique en C16 à C24, ledit acide et/ou ledit alcool étant en configuration trans, et d'au moins une cire hydrocarbonée de polyéthylène de point de fusion supérieur à 70 °C, dans une composition cosmétique pour améliorer son confort, son glissant à l'application et son onctuosité tout en préservant ses propriétés mécaniques.

La composition selon l'invention comprend au moins un ester d'un acide éthylénique en C16 à C24 et d'un alcool éthylénique en C16 à C24 avec ledit acide et/ou ledit alcool étant en configuration trans.

Selon le mode de mise en oeuvre, la composition est solide, en ce sens qu'elle présente une dureté particulière. La dureté de la composition peut être mesurée par la méthode dite du fil à couper le beurre, qui consiste à couper un stick de composition à tester, de diamètre compris entre 8 et 12,7 mm et à mesurer la dureté à 20 °C, au moyen d'un dynamomètre DFGHS 2 de la société Indelco-Chatillon se déplaçant à une vitesse de 100mm/minute. Elle est exprimée comme la force de cisaillement (exprimée en gramme) nécessaire pour couper un stick dans ces conditions. Selon cette méthode, la dureté de la composition selon l'invention varie de 40 à 250 g, en particulier de 60 à 190 g et plus particulièrement de 80 à 170 g.

Au sens de la présente invention, un acide éthylénique en C16 à C24 désigne un composé hydrocarboné en C16 à C24 comportant au moins une insaturation éthylénique et au moins une fonction carboxylique.

On entend par "composé hydrocarboné", un composé constitué essentiellement de carbone et d'hydrogène et éventuellement fonctionnalisé par un groupe comprenant un hétéroatome, comme l'oxygène, l'azote ou un halogène.

Selon un mode de réalisation particulier, ledit acide est exempt de tout groupe fonctionnalisé autre que la fonction carboxyle. En particulier, il est exempt de groupe hydroxyle.

L'acide éthylénique peut présenter une seule fonction carboxylique ou plusieurs fonctions carboxyliques. Avantageusement, il en porte une seule.

L'acide éthylénique conforme à l'invention, peut être linéaire ou ramifié. Il est en particulier linéaire.

Ledit acide comprend au moins une insaturation éthylénique. Il comprend en particulier une seule insaturation éthylénique.

L'acide éthylénique comprend de 16 à 24 atomes de carbone en particulier de 20 à 22 atomes de carbone.

A titre représentatif du type d'acide convenant à l'invention, on peut particulièrement citer les composés de formule comme suit :

CH₃-(CH₂)n-CH=CH-(CH₂)ₘ-COOH

dans laquelle la somme de n et m varie de 14 à 20, n et m désignant des entiers naturels. En particulier m varie de 8 à 12.

Par exemple, l'acide éthylénique est choisi dans le groupe constitué par l'acide octadéc-9-énoïque ou acide oléique (C₁₈), l'acide eicos-11-énoique (C₂₀), l'acide docos-13-énoique ou acide érucique (C₂₂) tel que n=7 et m=11, l'acide tétracos-15-énoique (C₂₄) et leurs mélanges.

Au sens de la présente invention, un alcool éthylénique en C₁₆ à C₂₄ désigne un composé hydrocarboné en C₁₆ à C₂₄, comportant au moins une insaturation éthylénique et au moins une fonction hydroxyle.

Cet alcool peut présenter une seule fonction hydroxyle ou plusieurs fonctions hydroxyles. Avantageusement, il en porte une seule.

Selon un mode de réalisation particulier, ledit alcool est exempt de tout groupe fonctionnalisé autre que la fonction hydroxyle.

L'alcool conforme à l'invention peut être ramifié ou linéaire. Il est en particulier linéaire.

Ledit alcool comprend au moins une insaturation éthylénique. Il comprend en particulier une seule insaturation éthylénique.

L'alcool éthylénique conforme à l'invention peut être notamment choisi parmi les alcools en C₂₀ et en C₂₂.

Selon un mode de réalisation particulier, il répond à la formule générale suivante :

CH₃-(CH₂)ᵣ-CH=CH-(CH₂)ₛ-OH

dans laquelle la somme de r et s varie de 15 à 21, r et désignant des entiers naturels. En particulier, s varie de 10 à 14.

A titre illustratif des alcools convenant à l'invention, on peut particulièrement citer l'octadéc-9-énol (C₁₈), l' eicos-11-énol (C₂₀), le docos-13-énol (C₂₂), le tétracos-15-énol (C₂₄), ou un de leurs mélanges.

Comme précisé précédemment, l'ester selon l'invention possède au moins une insaturation éthylénique ou liaison éthylénique en configuration trans, soit sur le radical alcoyle, soit sur le radical alcanoyle de la molécule. Selon une variante particulière de l'invention, l'ester est doté de deux liaisons éthyléniques en configuration trans, l'une située sur le radical alcoyle et l'autre située sur le radical alcanoyle de la molécule.

Selon un mode de réalisation particulier de l'invention, l'ester comprend de 38 à 44 atomes de carbone. Il peut être choisi en particulier dans le groupe constitué par l'oléate d'eicos-11-ényle, l'eicos-11-énoate d'eicos-11-ényle, l'eicos-11-énoate de docos-13-ényle, l'eicos-11-énoate de tétracos-15-ényle, le docos-13-énoate d'eicos-11-énoyle et leurs mélanges.

Plus particulièrement, la composition de l'invention contient l'ester tel que défini précédemment sous la forme d'un mélange.

Selon un mode de réalisation particulier de l'invention, ledit ester est introduit dans la composition sous la forme de beurre de jojoba.

Le beurre de jojoba peut être obtenu à partir d'huile de jojoba, extraite de la graine de l'espèce *Simmondsia chinensis,* par exemple selon le procédé décrit dans le brevet US 4 329 298.

Conviennent ainsi à l'invention, notamment les beurres de jojoba commercialisés sous les dénominations « ISO-jojoba 35® » et « ISO-jojoba 50® » par la société DESERT WHALE JOJOBA COMPANY.

On choisit en particulier un beurre de jojoba dont la température de fusion est inférieure à 60 °C, notamment allant de 35 à 50 °C. Le point de fusion est mesuré au sommet du pic le plus endotherme du thermogramme tracé à l'aide d'un calorimètre à balayage différentiel (D. S. C), tel que le calorimètre vendu sous la dénomination « MDSC 2920® » par la société TA instrument, avec une montée en température de 5 ou 10 °C par minute, selon la norme ISO 11357-3:1999.

Le beurre de jojoba est avantageusement présent dans la composition selon l'invention en une teneur allant de 0,1 à 40 %, par exemple de 0,5 à 15 %, en particulier de 1 à 12 % et plus particulièrement de 1,5 à 8 % en poids. Pour des raisons évidentes, cette proportion est bien entendue susceptible de varier significativement selon la nature de la composition cosmétique considérée.

La composition selon l'invention contient en outre au moins une cire hydrocarbonée de polyéthylène ayant un point de fusion supérieur à 70 °C.

Par "cire" au sens de la présente invention, on entend un composé gras lipophile, solide à température ambiante (25 °C), à changement d'état solide/liquide réversible, ayant une température de fusion supérieure ou égale à 30 °C pouvant aller jusqu'à 200 °C, une dureté supérieure à 0,5 MPa, et présentant à l'état solide une organisation cristalline anisotrope.

On entend par cire hydrocarbonée, une cire constituée uniquement de carbone et d'hydrogène.

La cire hydrocarbonée conforme à l'invention est choisie parmi les cires de polyéthylène, sous réserve que ladite cire présente un point de fusion mesuré selon la méthode décrite précédemment supérieur à 70 °C.

La cire ayant un point de fusion supérieur à 70 °C peut être présente dans les compositions selon l'invention à raison de 0,1 à 25 % en poids, notamment de 2 à 20 % en poids, en particulier de 3 à 15 %, et plus particulièrement de 5 à 12 % en poids par rapport au poids total de la composition.

La cire hydrocarbonée conforme à l'invention a en particulier un point de fusion inférieur ou égal à 105 °C, en particulier allant de 75 °C à 100 °C, et plus particulièrement de 80 à 95 °C.

Selon un mode de réalisation particulier, la composition selon l'invention comprend au moins deux cires hydrocarbonées différentes.

Dans une première variante, ces cires se distinguent par leur structure, les unes étant linéaires et les autres ramifiées. Ainsi, la composition comprend avantageusement le mélange d'au moins une cire hydrocarbonée linéaire et d'au moins une cire hydrocarbonée ramifiée. Il peut s'agir en particulier d'un mélange d'au moins une cire de polyéthylène et d'au moins une cire microcristalline.

Dans une seconde variante, la composition selon l'invention comprend un mélange d'au moins deux cires hydrocarbonées ayant des points de fusion différents. Les compositions selon l'invention peuvent en particulier comprendre un mélange d'au moins une cire ayant un point de fusion supérieur à 70 °C et pouvant aller jusqu'à 80 °C inclus et d'au moins une cire ayant un point de fusion supérieur à 80 °C et en particulier inférieur ou égal à 105°C.

Avantageusement, le rapport pondéral cire ayant un point de fusion supérieur à 70 °C et inférieur ou égal à 80 °C sur cire ayant un point de fusion supérieur à 80 °C est de 1/1 à 3/1, et en particulier d'environ 2/1.

Ces cires se distinguant par leur point de fusion peuvent également se distinguer par leur structure, l'une pouvant être de type linéaire et l'autre de type ramifié. De plus, la composition selon l'invention peut comprendre un mélange de cires de points de fusion différents comme défini précédemment, avec pour une gamme de point de fusion donnée ou pour un point de fusion donné, un mélange de cires qui présentent des structures différentes, de type linéaire et ramifié.

Au sens de la présente invention, on entend par l'expression cire de polyéthylène, un polymère choisi parmi les homopolymères d'éthylène et les copolymères d'éthylène et d'un monomère répondant à la formule :

CH₂=CH-R'

dans laquelle :
R' représente un radical alkyle ayant de 1 à 30 atomes de carbone, un radical aryle ou aralkyle.

Par polymère, on entend un composé comportant au moins 2 motifs de répétition, en particulier au moins 3 motifs de répétition et plus particulièrement au moins 10 motifs de répétition.

Parmi les radicaux alkyles ayant de 1 à 30 atomes de carbone, on peut citer les radicaux méthyle, éthyle, propyle, isopropyle, décyle, dodécyle et octadécyle.

Le radical aryle est en particulier le radical phényle ou tolyle.

Le radical aralkyle est en particulier le radical benzyle ou phénéthyle.

Ces cires étant des produits de synthèse, elles ne présentent pas les problèmes de variabilité observée avec les composés naturels.

Selon un mode de réalisation particulier des compositions selon l'invention, la cire utilisée telle que définie précédemment est choisie parmi les homopolymères d'éthylène, les copolymères éthylène-propylène et les copolymères éthylène-hexène, et avantageusement parmi les homopolymères d'éthylène.

Avantageusement, la cire de polyéthylène utilisée dans les compositions de l'invention présente un point de fusion mesuré selon la méthode de calorimétrie décrite précédemment, supérieur à 70 °C et inférieur ou égal à 105 °C, en particulier allant de 80 à 95 °C. Selon un mode de réalisation particulier des compositions selon l'invention, les cires de polyéthylène sont choisies parmi les polymères, et notamment les homopolymères, ayant un poids moléculaire allant de 300 à 700, en particulier de 350 à 650.

Comme homopolymères d'éthylène utilisables dans les compositions selon l'invention, on peut citer notamment les cires commercialisées sous les dénominations de « Performalen 500® » et « 400® » par la société NEWPHASE TECHNOLOGIES ayant respectivement des poids moléculaires de 500 et 400.

Parmi les copolymères d'éthylène utilisables selon l'invention, on peut citer notamment les copolymères éthylène-propylène commercialisés sous la dénomination de « Performalen EP 700® » par la société NEWPHASE TECHNOLOGIES.

Selon une variante de l'invention, on peut avantageusement utiliser un mélange de cire de polyéthylène présentant des points de fusion différents. On peut en particulier utiliser un mélange d'au moins une cire de polyéthylène ayant un point de fusion supérieur à 70 °C et inférieur ou égal à 80 °C et d'au moins une cire de polyéthylène ayant un point de fusion supérieur à 80 °C et inférieur ou égal à 105 °C, lesdites cires pouvant être notamment présentes en un rapport pondéral allant de 1/1 à 3/1, et en particulier en un rapport pondéral d'environ 2/1. Avantageusement, la composition selon l'invention comprend un mélange d'au moins une cire de polyéthylène ayant un point de fusion inférieur ou égal à 75 °C et d'au moins une cire de polyéthylène ayant un point de fusion supérieur à 80 et inférieur ou égal à 90 °C.

De manière avantageuse, l'ester et la cire requis dans le cadre de la présente invention sont associés dans la composition cosmétique dans un rapport pondéral ester/cire généralement inférieur à 1, et notamment pouvant aller de 0,15 à 0,75.

Selon une variante, la composition conforme à l'invention comprend de 0,1 à 40 % de beurre de jojoba et de 0,1 à 25 % de cire hydrocarbonée, en particulier de 0,5 à 15 % de beurre de jojoba et de 2 à 20 % de cire, et plus particulièrement de 1 % à 12 % de beurre de jojoba et de 3 à 15 % de cire, lesdits pourcentages étant exprimés en poids par rapport au poids total de la composition.

Bien entendu d'autres additifs cosmétiques peuvent être associés à la cire et l'ester selon l'invention de manière à conférer à la composition cosmétique considérée les propriétés requises notamment en terme de point de fusion, de fluidité, et de résistance mécanique.

Ainsi, les compositions selon l'invention, notamment lorsqu'elles sont destinées à être appliquées sur les lèvres, la peau et les cils, peuvent comporter un corps gras supplémentaire différent de l'ester et de la cire décrits précédemment, notamment au moins un corps gras liquide à température ambiante (25 °C) et à pression atmosphérique.

La composition peut posséder par exemple une phase grasse continue, pouvant contenir moins de 5 % d'eau, notamment moins de 1 % d'eau par rapport à son poids total et en particulier peut être sous forme anhydre.

Ladite phase grasse peut notamment comprendre, à titre de corps gras liquide, au moins une huile volatile ou non volatile ou un de leurs mélanges.

Par « huile volatile », on entend au sens de l'invention toute huile susceptible de s'évaporer au contact de la peau en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40.000 Pa) et plus particulièrement supérieure à 0,3 mm de Hg (30 Pa).

Par « huile non volatile », on entend une huile ayant notamment une pression de vapeur inférieure à 0,01 mm de Hg (1,33 Pa).

Ces huiles volatiles ou non volatiles peuvent être des huiles hydrocarbonées notamment d'origine végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permetyls® , les esters ramifiés en C₈-C₁₆ tels que le néopentanoate d'iso-hexyle, et leurs mélanges. D'autres huiles hydrocarbonées volatiles comme les distillats de pétrole, notamment ceux vendus sous la dénomination Shell Solt® par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 8 centistokes (8 × 10-6 m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans la composition selon l'invention à une teneur allant de 0,01 à 50 % en poids, notamment de 0,1 à 40 % en poids, et en particulier de 1 à 30 % en poids, par rapport au poids total de la composition.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de luzerne, de pavot, de potimarron, de sésame, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810® , 812® et 818® par la société DYNAMIT NOBEL,
- les éthers de synthèse ayant de 10 à 40 atomes de carbone ; les hydrocarbures linéaires ou ramifiés, d'origine minérale ou synthétique tels que la vaseline, les polydécènes, le polyisobutène hydrogéné tel que le Parléam® , le squalane et leurs mélanges,
- les esters de synthèse comme les huiles de formule R¹COOR² dans laquelle R¹ représente le reste d'un acide gras linéaire ou ramifié comportant de 1 à 40 atomes de carbone et R² représente une chaîne hydrocarbonée notamment ramifiée contenant de 1 à 40 atomes de carbone à condition que R¹ + R² soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; le polylaurate de vinyle; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates.

Les huiles non volatiles peuvent être présentes dans la composition selon l'invention en une teneur allant de 0,01 à 90 % en poids, notamment de 0,1 % à 85 % en poids, et en particulier de 1 % à 70 % en poids, par rapport au poids total de la composition.

Selon un autre mode de réalisation particulier, la composition conforme à l'invention contient au moins une huile de silicone et/ou une huile hydrocarbonée apolaire.

Par « huile hydrocarbonée apolaire », on entend une huile constituée uniquement de carbone et d'hydrogène. Une telle huile hydrocarbonée apolaire est donc exempte de groupe fonctionnel polaire tel que des groupes hydroxyles ou carboxyles et d'hétéroatome. Une telle huile est en particulier choisie parmi la vaseline, les polydécènes, les polydécènes hydrogénés, le squalane, le polybutène, le polyisobutène, le polyisobutène hydrogéné ou un de leurs mélanges.

La composition contient en particulier au moins 20 %, et plus particulièrement au moins 30 % en poids par rapport au poids total de la composition d'une huile siliconée et/ou d'une huile hydrocarbonée apolaire telle que décrite précédemment.

La composition selon l'invention contient notamment moins de 40 %, en particulier moins de 30 %, et plus particulièrement moins de 20 % en poids par rapport au poids total de la composition, d'une huile hydrocarbonée polaire et en particulier d'une huile hydrocarbonée comportant des fonctions hydroxyles.

La composition selon l'invention est en particulier exempte d'huile de ricin.

Plus généralement, le corps gras liquide à température ambiante et à pression atmosphérique peut être présent à raison de 0,01 à 90 % en poids et notamment de 0,1 à 85 % en poids, par rapport au poids de la phase grasse.

En ce qui concerne le corps gras supplémentaire solide à température ambiante et à pression atmosphérique différent de l'ester et de la cire ayant un point de fusion supérieur à 70 °C décrits précédemment, il peut être choisi parmi les cires ayant un point de fusion inférieur ou égal à 70 °C, les corps gras pâteux, les gommes et leurs mélanges. Ce corps gras solide peut être présent à raison de 0,01 à 50 %, notamment de 0,1 à 40 % et en particulier de 0,2 à 30 % en poids, par rapport au poids total de la phase grasse.

Selon un mode de réalisation de l'invention, la composition peut comprendre, outre la cire ayant un point de fusion supérieur à 70 °C, une cire supplémentaire. Elle peut être hydrocarbonée, fluorée et/ou siliconée et être d'origine animale, végétale, minérale ou synthétique. Elle peut être choisie par exemple parmi la cire d'abeille, la cire de Carnauba, la cire de Candellila et leurs mélanges.

Selon un autre mode de réalisation, la composition selon l'invention est exempte de cire ayant un point de fusion inférieur ou égal à 70 °C. En particulier, la composition selon l'invention est exempte d'ozokérite.

Selon une variante de l'invention, la composition peut comprendre au moins un composé gras pâteux à température ambiante.

Par "composé pâteux" au sens de la présente invention, on entend désigner un composé gras lipophile, à changement d'état solide/liquide réversible présentant à l'état solide une organisation cristalline anisotrope, et comportant à la température de 23 °C une fraction liquide et une fraction solide.

Par composé pâteux au sens de l'invention, on entend un composé ayant une dureté à 20 °C allant de 0,001 à 0,5 MPa, et en particulier allant de 0,002 à 0,4 MPa.

La dureté est mesurée selon une méthode de pénétration d'une sonde dans un échantillon de composé et en particulier à l'aide d'un analyseur de texture (par exemple le TA-XT2i de chez Rhéo) équipé d'un cylindre en inox de 2 mm de diamètre. La mesure de dureté est effectuée à 20 °C au centre de 5 échantillons. Le cylindre est introduit dans chaque échantillon à une pré-vitesse de 1mm/s puis à une vitesse de mesure de 0,1 mm/s, la profondeur de pénétration étant de 0,3 mm. La valeur relevée de la dureté est celle du pic maximum.

Ce composé pâteux est en outre, à la température de 23 °C, sous la forme d'une fraction liquide et d'une fraction solide. En d'autres termes, la température de fusion commençante du composé pâteux est inférieure à 23 °C. La fraction liquide du composé pâteux mesurée à 23 °C représente 9 à 97 % en poids du composé. Cette fraction liquide à 23 °C représente en particulier de 15 à 85 %, et plus particulièrement de 40 à 85 % en poids.

La fraction liquide en poids du composé pâteux à 23 °C est égale au rapport de l'enthalpie de fusion consommée à 23 °C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920 par la société TA instrument, avec une montée en température de 5 ou 10 °C par minute, selon la norme ISO 11357-3:1999. L'enthalpie de fusion du composé pâteux est la quantité d'énergie nécessaire pour faire passer le composé de l'état solide à l'état liquide. Elle est exprimée en J/g.

L'enthalpie de fusion consommée à 23 °C est la quantité d'énergie absorbée par l'échantillon pour passer de l'état solide à l'état qu'il présente à 23 °C constitué d'une fraction liquide et d'une fraction solide.

La fraction liquide du composé pâteux mesurée à 32 °C représente en particulier de 30 à 100 % en poids du composé, plus particulièrement de 80 à 100 %, voire de 90 à 100 % en poids du composé. Lorsque la fraction liquide du composé pâteux mesurée à 32 °C est égale à 100 %, la température de la fin de la plage de fusion du composé pâteux est inférieure ou égale à 32 °C.

La fraction liquide du composé pâteux mesurée à 32 °C est égale au rapport de l'enthalpie de fusion consommée à 32 °C sur l'enthalpie de fusion du composé pâteux. L'enthalpie de fusion consommée à 32 °C est calculée de la même façon que l'enthalpie de fusion consommée à 23 °C.

Le composé pâteux est avantageusement choisi parmi :
- les composés siliconés polymères ou non,
- les composés fluorés polymères ou non,
- les polymères vinyliques, notamment :
   - les homopolymères d'oléfmes
   - les copolymères d'oléfines
   - les homopolymères et copolymères de diènes hydrogénés
   - les oligomères linéaires ou ramifiés, homo ou copolymères de (méth)acrylates d'alkyles ayant de préférence un groupement alkyle en C₈-C₃₀
   - les oligomères homo et copolymères d'esters vinyliques ayant des groupements alkyles en C₈-C₃₀
   - les oligomères homo et copolymères de vinyléthers ayant des groupements alkyles en C₈-C₃₀,
- les polyéthers liposolubles résultant de la polyéthérification entre un ou plusieurs diols en C2-C100, en particulier en C2-C50,
- les esters,
- et leurs mélanges.

Le composé pâteux est en particulier de type polymère, notamment hydrocarboné.

La composition de l'invention peut en outre comprendre une phase particulaire pouvant être présente à raison de 0,01 à 40 % en poids, notamment de 0,01 à 30 % en poids et en particulier de 0,05 à 20 % en poids, par rapport au poids total de la composition.

Elle peut notamment comprendre des colorants et/ou des pigments et/ou des charges complémentaires utilisés dans les compositions cosmétiques.

Par pigments, il faut comprendre des particules blanches ou colorées, minérales ou organiques, destinées à colorer et/ou opacifier la composition. Les pigments incluent les nacres qui sont des particules irisées, notamment produites par certains mollusques dans leur coquille ou bien synthétisées.

Par charges, il faut comprendre des particules incolores ou blanches, minérales ou de synthèse, lamellaires ou non lamellaires.

Les pigments peuvent être présents dans la composition à raison de 0,01 à 25 % en poids, en particulier de 0,01 à 15 % en poids.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome. Parmi les pigments organiques utilisables dans l'invention, on peut citer le noir de carbone, les pigments de type D & C, et les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges complémentaires peuvent être présentes à raison de 0,01 à 40 % en poids, notamment 0,01 à 30 % en poids, et en particulier de 0,02 à 20 % en poids par rapport au poids total de la composition.

Il peut notamment s'agir de charges sphériques comme par exemple le talc, le stéarate de zinc, le mica, le kaolin, les poudres de polyamide (Nylon®) (Orgasol® de chez ATOCHEM), les poudres de polyéthylène, les poudres de polymères de tétrafluoroéthylène (Téflon® ), l'amidon, le nitrure de bore, des microsphères polymériques telles que celles de chlorure de polyvinylidène/acrylonitrile comme l'Expancel® (NOBEL INDUSTRIES), de copolymères d'acide acrylique (Polytrap® de la société DOW CORNING), les microbilles de résine de silicone (Tospearls® de TOSHIBA, par exemple), et les organopolysiloxanes élastomères.

La composition peut comprendre également des colorants hydrosolubles ou liposolubles en une teneur allant de 0,01 à 6 % en poids, par rapport au poids total de la composition, notamment allant de 0,01 à 3 % en poids. Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, et le jaune quinoléine. Les colorants hydrosolubles sont par exemple le jus de betterave et le bleu de méthylène.

La composition selon l'invention peut, de plus, comprendre tous les ingrédients classiquement utilisés dans les domaines concernés et plus spécialement dans le domaine du maquillage et du soin. Ces ingrédients sont en particulier choisis parmi les vitamines, les antioxydants, les épaississants, les oligo-éléments, les adoucissants, les séquestrants, les parfums, les agents alcalinisants ou acidifiants, les conservateurs, les filtres UV, les actifs hydrophiles ou lipophiles et leurs mélanges. Les quantités de ces différents ingrédients sont celles classiquement utilisées dans les domaines concernés et par exemple de 0,01 à 20 % du poids total de la composition.

Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction considérée.

La composition de l'invention peut être obtenue selon les procédés de préparation classiquement utilisés en cosmétique.

Elle se présente sous la forme d'un produit solide tel que sous la forme d'un produit coulé en stick ou en coupelle comme les rouges à lèvres ou les baumes à lèvres, les fonds de teint coulés, les produits anti-cemes, les « correcteurs » et/ou « embellisseurs » de teint et les fards à paupières ou à joues.

Elle peut se présenter sous la forme d'un produit de soin et/ou de maquillage des ongles, de la peau et/ou des lèvres.

Elle peut également se présenter sous la forme d'un produit de maquillage des cils. Les mascaras ainsi obtenus peuvent notamment se présenter sous la forme d'un pain.

La présente invention concerne également un procédé de traitement cosmétique d'au moins une matière kératinique, notamment la peau, les cheveux et/ou les ongles comprenant l'application sur ladite matière d'une composition selon l'invention.

Les exemples de composition ci-après sont donnés à titre illustratif.

### Exemple 1

On a préparé la composition de rouge à lèvres suivante (pourcentages massiques) :
- Cire de Polyéthylène (PM = 500) (« Performalen 500® »
   de NEW PHASE TECHNOLOGIES) 10
- Cire microcristalline (« Microwax HW® » de PARAMELT) 2,9
- Beurre de jojoba (« ISO-Jojoba 50® » de DESERT WHALE JOJOBA COMPANY) 2,5
- Silice enrobée diméthicone (« SA-SB-300® » de MIYOSHI) 4
- Pigments 8,66
- Polybutène (« Indopol H-100® » d'AMOCO) 5
- Trimellitate de tridécyle 9
- Malate de diisostéaryle 10
- Polyisobutène hydrogéné (965 g/mol) 24,54
- Phényltriméthicone (« DC 556® » de DOW CORNING) 10
- Polylaurate de vinyle 13,4

La composition de rouge à lèvres de l'exemple 1 montre de bonnes propriétés d'application (en termes de fondant, de glissant et de douceur) ainsi qu'une excellente stabilité dans le temps. Elle résiste à l'écrasement sur le dos de la main après conservation pendant 24 heures dans une étuve contrôlée à 34, 36 ou même 38 °C.

### Exemple 2

On a préparé le bâton de rouge à lèvres suivant (pourcentages massiques)
- Octyl-2 dodécanol 20
- Cire microcristalline (« Microwax HW® » de PARAMELT) 3
- Triglycérides d'acides laurique / palmitique / cétylique /
   stéarique (50/20/10/10) («Softisan 100® » de SASOL) 5
- Phényltriméthylsiloxytrisiloxane (« DC 556® » de
   DOW CORNING) 6
- Copolymère méthacrylate de lauryle / diméthacrylate
   d'éthylène glycol («Polytrap 6603® » de RP SCHERER) 0,5
- Cire de polyéthylène (« Performalen 500® » de la société
   NEW PHASE TECHNOLOGIES) 8
- Mélange de glycérides d'acides gras végétaux / acide
   isostéarique / acide adipique (« Softisan 649® » de SASOL) 13
- Malate de diisostéaryle 18
- Tétra-isostéarate de pentaérythrityle 14
- Beurre de jojoba
   (« ISO-Jojoba 50® » de DESERT WHALE JOJOBA COMPANY) 2,55
- Pigments qsp 100

Cette composition dispose de bonnes propriétés d'application. Elle confère de la brillance à un niveau satisfaisant aussi bien juste après son application qu'au terme d'une heure. Elle est confortable dans le temps et est dénuée d'effet collant

Elle possède une bonne stabilité dans le temps et elle n'est pas sujette à des problèmes de cristallisation, ni d'exsudation.

Elle présente également une bonne résistance à l'écrasement à 38 °C.

## Revendications

1. Composition cosmétique solide, notamment pour le soin et/ou le maquillage de la peau, des lèvres et/ou des matières kératiniques **caractérisée en ce qu'**elle associe à au moins un ester d'un acide éthylénique en C₁₆ à C₂₄ et d'un alcool éthylénique en C₁₆ à C₂₄, ledit acide et/ou ledit alcool étant en configuration trans, au moins une cire hydrocarbonée de polyéthylène de point de fusion supérieur à 70 °C.

2. Composition selon la revendication 1, **caractérisée en ce que** ledit acide ne comporte aucun groupe hydroxyle.

3. Composition selon l'une quelconque des revendications précédentes, **caractérisée en ce que** ledit acide est choisi parmi les acides en C₂₀ et en C₂₂.

4. Composition selon l'une quelconque des revendications précédentes, **caractérisée en ce que** ledit acide est linéaire.

5. Composition selon l'une quelconque des revendications précédentes, **caractérisée en ce que** ledit acide comporte une seule insaturation éthylénique.

6. Composition selon l'une des revendications 1 et 2, **caractérisée en ce que** ledit acide est choisi dans le groupe constitué par l'acide octadéc-9-énoïque ou acide oléique (C₁₈), l'acide eicos-11-énoique (C₂₀), l'acide docos-13-énoique ou acide érucique (C₂₂), l'acide tétracos-15-énoique (C₂₄) et leurs mélanges.

7. Composition selon l'une quelconque des revendications précédentes, **caractérisée en ce que** ledit alcool est choisi parmi les alcools en C₂₀ et en C₂₂.

8. Composition selon l'une quelconque des revendications précédentes, **caractérisée en ce que** ledit alcool est linéaire.

9. Composition selon l'une quelconque des revendications précédentes, **caractérisée en ce que** ledit alcool comporte une seule insaturation éthylénique.

10. Composition selon l'une quelconque des revendications 1 à 6, **caractérisée en ce que** ledit alcool est choisi parmi l'octadéc-9-énol (C₁₈), l'eicos-11-énol (C₂₀), le docos-13-énol (C₂₂), le tétracos-15-énol (C₂₄), et leurs mélanges.

11. Composition selon l'une quelconque des revendications précédentes, **caractérisée en ce que** ledit ester est choisi dans le groupe constitué par l'oléate d'eicos-11-ényle, l'eicos-11-énoate d'eicos-11-ényle, l'eicos-11-énoate de docos-13-ényle, l'eicos-11-énoate de tétracos-15-ényle, le docos-13-énoate d'eicos-11-énoyle et leurs mélanges.

12. Composition selon l'une quelconque des revendications précédentes, **caractérisée en ce que** ledit ester est introduit dans la composition sous la forme de beurre de jojoba.

13. Composition selon la revendication 12, **caractérisée en ce que** le beurre de jojoba est présent dans la composition selon l'invention en une teneur allant de 0,1 à 40 %, notamment de 0,5 à 15 %, en particulier de 1 à 12 % et plus particulièrement de 1,5 à 8 % en poids par rapport au poids total de la composition.

14. Composition selon l'une quelconque des revendications précédentes, **caractérisée en ce que** la cire a un point de fusion inférieur ou égal à 105 °C, en particulier allant de 75 °C à 100 °C, et plus particulièrement de 80 à 95 °C.

15. Composition selon la revendication 1, **caractérisée en ce qu'**elle comprend au moins deux cires hydrocarbonées différentes.

16. Composition selon la revendication 15, **caractérisée en ce qu'**elle comprend un mélange d'au moins une cire hydrocarbonée linéaire et d'au moins une cire hydrocarbonée ramifiée.

17. Composition selon la revendication 16, **caractérisée en ce que** ledit mélange est constitué d'au moins une cire de polyéthylène et d'au moins une cire microcristalline.

18. Composition selon l'une des revendications précédentes, **caractérisée en ce qu'**elle comprend un mélange d'au moins une cire hydrocarbonée ayant un point de fusion supérieur à 70 °C et inférieur ou égal à 80 °C et d'au moins une cire ayant un point de fusion supérieur à 80 °C et en particulier inférieur ou égal à 105 °C.

19. Composition selon la revendication 18, **caractérisée en ce que** le rapport pondéral de la cire ayant un point de fusion supérieur à 70 °C et inférieur ou égal à 80 °C sur la cire ayant un point de fusion supérieur à 80 °C est de 1/1 à 3/1, et en particulier d'environ 2/1.

20. Composition selon l'une quelconque des revendications précédentes, **caractérisée en ce que** ladite cire de polyéthylène possède un poids moléculaire allant de 300 à 700, et en particulier allant de 350 à 650.

21. Composition selon l'une quelconque des revendications précédentes, **caractérisée en ce qu'**elle comprend un mélange d'au moins une cire de polyéthylène ayant un point de fusion supérieur à 70 °C et inférieur ou égal à 80 °C et d'au moins une cire de polyéthylène ayant un point de fusion supérieur à 80 °C et inférieur ou égal à 105 °C, lesdites cires étant en particulier respectivement présentes en un rapport pondéral allant de 1/1 à 3/1, et plus particulièrement d'environ 2/1.

22. Composition selon la revendication précédente, **caractérisée en ce qu'**elle comprend un mélange d'au moins une cire de polyéthylène ayant un point de fusion inférieur ou égal à 75 °C et d'au moins une cire de polyéthylène ayant un point de fusion supérieur à 80 °C et inférieur ou égal à 90 °C.

23. Composition selon l'une quelconque des revendications précédentes; **caractérisée en ce que** ladite cire est présente en une teneur allant de 0,1 à 25 %, notamment de 2 à 20 %, en particulier de 3 à 15 % et plus particulièrement de 5 à 12 % en poids par rapport au poids total de la composition.

24. Composition selon l'une quelconque des revendications précédentes, **caractérisée en ce qu'**elle comprend au moins une huile de silicone et/ou une huile hydrocarbonée apolaire.

25. Composition selon l'une quelconque des revendications précédentes, **caractérisée en ce qu'**elle est anhydre.

26. Composition selon l'une quelconque des revendications précédentes, **caractérisée en ce qu'**elle comprend au moins un colorant et/ou au moins un pigment et/ou au moins une charge complémentaire.

27. Utilisation de l'association d'au moins un ester d'un acide éthylénique en C₁₆ à C₂₄ et d'un alcool éthylénique en C₁₆ à C₂₄, ledit acide et/ou ledit alcool étant en configuration trans, et d'au moins une cire hydrocarbonée de polyéthylène de point de fusion supérieur à 70 °C, dans une composition cosmétique solide pour améliorer son confort, son glissant à l'application et son onctuosité tout en préservant ses propriétés mécaniques.

28. Utilisation selon la revendication 27, **caractérisée en ce que** ledit ester est tel que défini en revendications 2 à 13.

29. Utilisation selon la revendication 27 ou 28, **caractérisée en ce que** ladite cire est telle que définie en revendications 14 à 23.

## Claims

1. Solid cosmetic composition, in particular for caring for and/or making up the skin, lips and/or keratinous substances, **characterized in that** it combines, with at least one ester of a C₁₆ to C₂₄ ethylenic acid and of a C₁₆ to C₂₄ ethylenic alcohol, said acid and/or said alcohol being of trans configuration, at least one polyethylene hydrocarbon wax with a melting point of greater than 70°C,

2. Composition according to Claim 1, **characterized in that** said acid does not comprise any hydroxyl group.

3. Composition according to either one of the preceding claims, **characterized in that** said acid is chosen from C₂₀ and C₂₂ acids.

4. Composition according to any one of the preceding claims, **characterized in that** said acid is linear.

5. Composition according to any one of the preceding claims, **characterized in that** said acid comprises a single ethylenic unsaturation.

6. Composition according to either of Claims 1 and 2, **characterized in that** said acid is chosen from the group consisting of octadec-9-enoic acid or oleic acid (C₁₈), eicos-11-enoic acid (C₂₀), docos-13-enoic acid or erucic acid (C₂₂, tetracos-15-enoic acid (C₂₄) and their mixtures.

7. Composition according to any one of the preceding claims, **characterized in that** said alcohol is chosen from C₂₀ and C₂₂ alcohols.

8. Composition according to any one of the preceding claims, **characterized in that** said alcohol is linear.

9. Composition according to any one of the preceding claims, **characterized in that** said alcohol comprises a single ethylenic unsaturation.

10. Composition according to any one of Claims 1 to 6, **characterized in that** said alcohol is chosen from octadec-9-enol (C₁₈), eicos-11-enol (C₂₀), docos-13-enol (C₂₂), tetracos-15-enol (C₂₄) and their mixtures.

11. Composition according to any one of the preceding claims, **characterized in that** said ester is chosen from the group consisting of eicos-11-enyl oleate, eicos-11-enyl eicos-11-enoate, docos-13-enyl eicos-11-enoate, tetracos-15-enyl eicos-11-enoate, eicos-11-enyl docos-13-enoate and their mixtures.

12. Composition according to any one of the preceding claims, **characterized in that** said ester is introduced into the composition in the form of jojoba butter.

13. Composition according to Claim 12, **characterized in that** the jojoba butter is present in the composition according to the invention in a content ranging from 0.1 to 40%, in particular from 0.5 to 15%, especially from 1 to 12% and more particularly from 1.5 to 8% by weight, with respect to the total weight of the composition.

14. Composition according to any one of the preceding claims, **characterized in that** the wax has a melting point of less than or equal to 105°C, in particular ranging from 75°C to 100°C and more particularly from 80 to 95°C.

15. Composition according to Claim 1, **characterized in that** it comprises at least two different hydrocarbon waxes.

16. Composition according to Claim 15, **characterized in that** it comprises a mixture of at least one linear hydrocarbon wax and of at least one branched hydrocarbon wax.

17. Composition according to Claim 16, **characterized in that** said mixture is composed of at least one polyethylene wax and of at least one microcrystalline wax.

18. Composition according to one of the preceding claims, **characterized in that** it comprises a mixture of at least one hydrocarbon wax having a melting point of greater than 70°C and of less than or equal to 80°C and of at least one wax having a melting point of greater than 80°C and in particular of less than or equal to 105°C.

19. Composition according to Claim 18, **characterized in that** the ratio by weight of the wax having a melting point of greater than 70°C and of less than or equal to 80°C to the wax having a melting point of greater than 80°C is from 1/1 to 3/1 and in particular approximately 2/1.

20. Composition according to any one of the preceding claims, **characterized in that** said polyethylene wax has a molecular weight ranging from 300 to 700 and in particular ranging from 350 to 650.

21. Composition according to any one of the preceding claims, **characterized in that** it comprises a mixture of at least one polyethylene wax having a melting point of greater than 70°C and of less than or equal to 80°C and of at least one polyethylene wax having a melting point of greater than 80°C and of less than or equal to 105°C, said waxes being in particular respectively present in a ratio by weight ranging from 1/1 to 3/1 and more particularly of approximately 2/1.

22. Composition according to the preceding claim, **characterized in that** it comprises a mixture of at least one polyethylene wax having a melting point of less than or equal to 75°C and of at least one polyethylene wax having a melting point of greater than 80°C and of less than or equal to 90°C.

23. Composition according to any one of the preceding claims, **characterized in that** said wax is present in a content ranging from 0.1 to 25%, in particular from 2 to 20%, and especially from 3 to 15% and more particularly from 5 to 12% by weight, with respect to the total weight of the composition.

24. Composition according to any one of the preceding claims, **characterized in that** it comprises at least one silicone oil and/or one nonpolar hydrocarbon oil.

25. Composition according to any one of the preceding claims, **characterized in that** it is anhydrous.

26. Composition according to any one of the preceding claims, **characterized in that** it comprises at least one dye and/or at least one pigment and/or at least one additional filler.

27. Use of the combination of at least one ester of a C₁₆ to C₂₄ ethylenic acid and of a C₁₆ to C₂₄ ethylenic alcohol, said acid and/or said alcohol being of trans configuration, and of at least one polyethylene hydrocarbon wax with a melting point of greater than 70°C in a solid cosmetic composition for improving its comfort, its slip on application and its unctuousness while retaining its mechanical properties.

28. Use according to Claim 27, **characterized in that** said ester is as defined in Claims 2 to 13.

29. Use according to Claim 27 or 28, **characterized in that** said wax is as defined in Claims 14 to 23.

## Patentansprüche

1. Feste kosmetische Zusammensetzung, insbesondere für die Pflege und/oder Schminke der Haut, der Lippen und/oder der Keratinsubstanzen, **dadurch gekennzeichnet, dass** in ihr mindestens ein Ester einer ethylenischen C₁₆-C₂₄-Säure und ein ethylenischer C₁₆-C₂₄-Alkohol, wobei die Säure und/oder der Alkohol in trans-Komiguration vorliegt, und mindestens ein Polyethylen-Kohlenwasserstoffwachs mit einem Schmelzpunkt von größer ist als 70 °C kombiniert sind.

2. Zusammensetzung nach Anspruch 1, **dadurch gekennzeichnet, dass** die Säure keine Hydroxylgruppe einschließt.

3. Zusammensetzung nach einem der vorhergehenden Ansprüche, **dadurch gekennzeichnet, dass** die Säure aus C₂₀- und C₂₂- Säuren ausgewählt ist.

4. Zusammensetzung nach einem der vorhergehenden Ansprüche, **dadurch gekennzeichnet, dass** die Säure linear ist.

5. Zusammensetzung nach einem der vorhergehenden Ansprüche, **dadurch gekennzeichnet, dass** die Säure eine einzige ethylenische Ungesättigtheit einschließt.

6. Zusammensetzung nach einem der Ansprüche 1 und 2, **dadurch gekennzeichnet, dass** die Säure aus der Gruppe ausgewählt ist, bestehend aus Octa-dec-9-ensäure oder aus Oleinsäure (C₁₈), Eicos-11-ensäure (C₂₀), Docos-13-ensäure oder aus Erucasäure (C₂₂), Tetrakos-15-ensäure (C₂₄), und ihren Gemischen.

7. Zusammensetzung nach einem der vorhergehenden Ansprüche, **dadurch gekennzeichnet, dass** der Alkohol aus den C₂₀- und C₂₂-Alkoholen ausgewählt ist.

8. Zusammensetzung nach einem der vorhergehenden Ansprüche, **dadurch gekennzeichnet, dass** der Alkohol linear ist.

9. Zusammensetzung nach einem der vorhergehenden Ansprüche, **dadurch gekennzeichnet, dass** der Alkohol eine einzige ethylenische Ungesättigtheit einschließt.

10. Zusammensetzung nach einem der Ansprüche 1 bis 6, **dadurch** gekennzeichet, dass der Alkohol aus Octadec-9-enol (C₁₈), Eicos-11-enol (C₂₀), Docos-13-enol (C₂₂), Tetrakos-15-enol (C₂₄), und ihren Gemischen ausgewählt ist.

11. Zusammensetzung nach einem der vorhergehenden Ansprüche, **dadurch gekennzeichnet, dass** der Ester aus der Gruppe ausgewählt ist, bestehend aus Eicos-11-enyloleat, Eicos-11-enyl-eicos-11-enoat, Docos-13-enyl-eicos-11-enoat von, Tetrakos-15-enyl-eicos-11-enoat, Eicos-11-enoyi-docos-13-enoat, und ihren Gemischen.

12. Zusammensetzung nach einem der vorhergehenden Ansprüche, **dadurch gekennzeichnet, dass** der Ester in die Zusammensetzung in der Form von Jojobabutter eingebracht ist.

13. Zusammensetzung nach Anspruch 12, **dadurch gekennzeichnet, dass** die Jojobabutter in einer erfindungsgemäßen Zusammensetzung in einem Gehalt von 0,1 bis 40 Gew.-%, insbesondere von 0,5 bis 15 Gew.-%, insbesondere von 1 bis 12 Gew.-%, und spezieller von 1,5 bis 8 Gew.-%, bezogen auf das Gesamtgewicht, der Zusammensetzung vorhanden ist.

14. Zusammensetzung nach einem der vorhergehenden Ansprüche, **dadurch gekennzeichnet, dass** das Wachs einen Schmelzpunkt von kleiner oder gleich 105 °C, insbesondere von 75 bis 100°C, und spezieller von 80 bis 95 °C aufweist.

15. Zusammensetzung nach Anspruch 1, **dadurch gekennzeichnet, dass** sie mindestens zwei verschiedene Kohlenwasserstoffwachse einschließt.

16. Zusammensetzung nach Anspruch 15, **dadurch gekennzeichnet, dass** sie eine Mischung von mindestens einem linearen Kohlenwasserstoffwachs und mindestens einem verzweigten Kohlenwasserstoffwachs einschließt.

17. Zusammensetzung nach Anspruch 16, **dadurch gekennzeichnet, dass** die Mischung aus mindestens einem Polyethylenwachs und mindestens einem mikrokristallinen Wachs aufgebaut ist.

18. Zusammensetzung nach einem der vorhergehenden Ansprüche, **dadurch gekennzeichnet, dass** sie eine Mischung von mindestens einem Kohlenwwasserstoffwachs mit einem Schmelzpunkt von größer als 70 °C und kleiner oder gleich 80 °C, und mindestens ein Wachs mit einem Schmelzpunkt von größer als 80 °C und insbesondere kleiner oder gleich 105 °C einschließt.

19. Zusammensetzung nach Anspruch 18, **dadurch gekennzeichnet, dass** das Gewichtsverhältnis des Wachses mit einem Schmelzpunkt von größer als 70 °C und kleiner oder gleich 80 °C zu dem Wachs mit einem Schmelzpunkt von größer als 80 °C 1/1 bis 3/1, und insbesondere ungefähr 2/1 beträgt.

20. Zusammensetzung nach einem der vorhergehenden Ansprüche, **dadurch gekennzeichnet, dass** das Polyethylenwachs ein Molekulargewicht von 300 bis 700 und insbesondere von 350 bis 650 besitzt.

21. Zusammensetzung nach einem der vorhergehenden Ansprüche, **dadurch gekennzeichnet, dass** sie eine Mischung von mindestens einem Polyethylenwachs mit einem Schmelzpunkt von größer als 70 °C und kleiner oder gleich 80 °C und von mindestens einem Polyethylenwachs mit einem Schmelzpunkt von größer als 80 °C und kleiner oder gleich 105 °C aufweist, wobei die Wachse insbesondere jeweils in einem Gewichtsverhältnis von 1/1 bis 3/1 und insbesondere von ungefähr 2/1 vorhanden sind.

22. Zusammensetzung nach dem vorhergehenden Anspruch, **dadurch gekennzeichnet, dass** sie eine Mischung von mindestens einem Polyethylenwachs mit einem Schmelzpunkt von kleiner oder gleich 75 °C und von mindestens einem Polyethylenwachs mit einem Schmelzpunkt von größer als 80 °C und kleiner oder gleich 90 °C einschließt.

23. Zusammensetzung nach einem der vorhergehenden Ansprüche, **dadurch gekennzeichnet, dass** das Wachs in einem Gehalt von 0,1 bis 25 Gew.-%, insbesondere von 2 bis 20 Gew.-%, insbesondere von 3 bis 15 Gew.-%, und spezieller von 5 bis 12 Gew.-%, bezogen auf das Gesamtgewicht der Zusammensetzung, vorhanden ist.

24. Zusammensetzung nach einem der vorhergehenden Ansprüche, **dadurch gekennzeichnet, dass** sie mindestens ein Silikonöl und/oder ein apolares Kohlenwasserstofföl einschließt.

25. Zusammensetzung nach einem der vorhergehenden Ansprüche, **dadurch gekennzeichnet, dass** sie wasserfrei ist.

26. Zusammensetzung nach einem der vorhergehenden Ansprüche, **dadurch gekennzeichnet, dass** sie mindestens ein Farbmittel und/oder mindestens ein Pigment und/oder mindestens eine zusätzlichen Füllstoff einschließt.

27. Verwendung der Kombination von mindestens einem Ester einer ethylenischen C₁₆-C₂₄-Säure und von eines ethylenischen C₁₆-C₂₄-Alkohols, wobei die Säure und/oder der Alkohol in trans-Konfiguration vorliegen, und von mindestens einem Polyethylen-Kohlenwasserstoffwachs mit einem Schmelzpunkt von größer als 70°C in einer festen kosmetischen Zusammensetzung zur Verbesserung ihrer Gebrauchsfreundlichkeit, ihres Gleitens beim Auftragen und ihrer Onktuosität, alles jeweils unter Beibehaltung ihrer mechanischen Eigenschaften.

28. Verwendung nach Anspruch 27, **dadurch gekennzeichnet, dass** der Ester so ist wie in den Ansprüchen 2 bis 13 definiert.

29. Verwendung nach Anspruch 27 oder 28, **dadurch gekennzeichnet, dass** das Wachs so ist wie in den Ansprüchen 14 bis 23 definiert.
